# EUROPEAN PATENT APPLICATION

(11) **EP 2 527 428 A1**
(43) Date of publication of application: **28.11.2012**
(21) Application number: 11382174.8
(22) Date of filing: 26.05.2011
(51) Int. Cl.: C12N 5/0784

(54) **Tolerogenic dendritic cells and their use in cell therapy**

(71) Applicant: Hospital Clínic de Barcelona, 08036 Barcelona (ES); Centro de Investigación Biomédica en Red de Enfermedades Hepáticas y Digestivas (CIBEREHD), 08036 Barcelona (ES)
(72) Inventor: Panés Díaz, Julián, 08036 Barcelona (ES); Ricart Gómez, Elena, 08036 Barcelona (ES); Benítez Ribas, Daniel, 08036 Barcelona (ES)
(74) Representative: ABG Patentes, S.L.

(57) **Abstract**

The invention describes a method for obtaining tolerogenic dendritic cells (DCs) manipulated *in vitro* with immunosuppressants. The tolerogenic DCs obtained can be used in the treatment of immune-mediated inflammatory diseases.

## Description

### Field of the Invention

The present invention relates to a method for obtaining tolerogenic dendritic cells manipulated in vitro with immunosuppressants. The functional properties of these cells make them optimum for the treatment of immune-mediated inflammatory diseases.

### Background of the Invention

Immune-mediated inflammatory diseases (IMIDs) form a group of diseases the cause of which is unknown although they are characterized by incurable chronic inflammation which can be a result of, or is caused by a deregulation of normal immune response. The inappropriate immune responses against antigens themselves or non-pathogenic antigens cause a chronic inflammation process with progressive and irreparable damage of the affected organs. Illustrative examples of IMIDs include arthritis, psoriatic arthritis, Behcet's disease, Crohn's disease, inflammatory bowel disease, ankylosing spondylitis, psoriasis, etc.

Currently, there are no effective pharmacology treatments for a significant group of patients with IMIDs which generates a significant clinical and socio-economic deterioration with a reduction in the quality of life of these patients. In some cases surgical resection of the affected organ can be performed, but in other cases the localization or extension of the disease prevents contemplating the possibility of a surgery. These patients suffer from an active inflammatory process and they make up the mortality associated with the disease. Thus there is a clear need of new forms of treatment for patients representing about 30% of the cases who are resistant to conventional therapies.

Dendritic cells (DCs) are the most potent antigen presenting cells described and they participate in the innate immunity and the acquired immune response. Despite the fact that promising results have been obtained in cell therapy with immunogenic DCs, no test has taken advantage from its tolerogenic properties as eventual therapeutic application. DCs are involved significantly in the induction of tolerance against self-antigens or non pathogenic antigens. This capacity for generating tolerance stimulates their use as eventual innovative therapy for the treatment of IMIDs which aim in silencing the aggravated immune reactions.

The generation and application of immunogenic DCs for the treatment of tumors is a process established for years; however, to date, no study has used these cells to produce tolerance in clinical assays.

Despite the fact that there are various protocols for the generation of tolerogenic DCs, none of them meets the strict clinical applicability requirements. Said protocols for obtaining tolerogenic DCs *in vitro* include one protocol which comprises culturing the DCs in the presence of vitamin D3, interleukin 10 (IL-10) or rapamycin, and dexamethasone. Generally, no maturation factor is used in said protocols, and, therefore, the cells maintain their immature phenotype. In some cases the endotoxin (lipopolysaccharide (LPS)) has been used for activating DCs [Anderson AE. et al., J Leukoc Biol. 2009 Feb;85(2):243-50] but this component cannot be used in clinical applications. As an alternative, the use of monophosphoryl lipid A (MPLA) has been described as the substitute of LPS, although it is less potent [Harry RA. et al., Ann Rheum Dis. 2010 Nov;69(11):2042-50].

Therefore, there is still a need of developing new protocols which allow obtaining tolerogenic DCs with migration and antigen-presenting capacity, pre-requirements necessary for a satisfactory immunotherapy, potentially useful in the treatment of IMIDs.

### Summary of the Invention

The inventors of the present invention have developed a method for obtaining mature tolerogenic dendritic cells (DCs) from monocytes. Said cells forming an aspect of this invention have very potent tolerogenic properties and are capable of inhibiting the responses of T lymphocyteT lymphocytes and of generating a population of regulatory T lymphocytes. The DCs provided by this invention can be used in the treatment of IMIDs.

In a first aspect, the invention relates to a method for obtaining tolerogenic DCs which comprises:
a) culturing monocytes originating from a peripheral blood sample of a subject in a culture medium comprising a cytokine for a time period comprised between 2 and 3 days,
b) culturing the cells resulting from step a) in a culture medium comprising a cytokine, a fat soluble vitamin and a steroid glucocorticoid for a time period of 4 days to obtain mature DCs,
c) culturing said mature DCs resulting from step b) in a culture medium comprising a proinflammatory cytokine, and
d) isolating the tolerogenic DCs.

In another aspect, the invention relates to tolerogenic DCs that can be obtained according to the aforementioned method provided by this invention. In a particular embodiment, said tolerogenic DC is characterized in that it shows an increased level of CD14 marker in comparison with the level of said CD14 marker in a mature DC obtained in step b) of the method provided by this invention. In another particular embodiment, said tolerogenic DC is characterized in that it shows reduced levels of CD80, CD83 and/or CCR7 markers, preferably reduced levels of the 3 markers in comparison with the levels of said markers in a mature DC obtained in step b) of the method provided by this invention.

In another aspect, the invention relates to a tolerogenic DC population provided by this invention.

In another aspect, the invention relates to a cell composition wherein at least 50% of the cells are the tolerogenic DCs provided by this invention.

In another aspect, the invention relates to a pharmaceutical composition comprising at least one tolerogenic DC provided by this invention, or a DC population provided by this invention, or a cell composition provided by this invention and a pharmaceutically acceptable carrier.

In another aspect, the invention relates to the use of a tolerogenic DC provided by this invention, or a DC population provided by this invention, or a cell composition provided by this invention, or a pharmaceutical composition provided by this invention in the preparation of a medication for the treatment of an IMID.

In another aspect, the invention relates to a tolerogenic DC provided by this invention, or a DC population provided by this invention, or a cell composition provided by this invention, or a pharmaceutical composition provided by this invention for use thereof in the treatment of an IMID.

### Brief Description of the Drawings

Figure 1 shows an illustrative scheme for preparing and obtaining tolerogenic DCs according to the present invention.
Figure 2 shows the results of the analysis by means of flow cytometry of CD14, CD80, CD83, CD86, CCR7, MHCI and MHCII markers in immature dendritic cells (iDCs), mature dendritic cells (mDCs) and tolerogenic dendritic cells (Tol-DCs).
Figure 3 is a graph showing the levels of IL-10 (pg/ml) of the cells kept in the presence of dexamethasone and vitamin A.
Figure 4 is a graph showing the result of the in vitro test wherein the proliferation of T lymphocytes in response to DCs of another donor is measured.

### Detailed Description of the Invention

### Definitions

In order to facilitate the understanding of the present invention, some terms and expressions used in the context of this invention are detailed below.

As used herein, the term "to isolate" is applied to a cell meaning that said cell (having a determined phenotype) or a cell population comprising said cells essentially lacks (is free) of other cells having a different phenotype which maybe present in a cell composition including said types of cells. Generally, a cell or a cell population, essentially free of other cells with a different phenotype when it is separated from, at least 50%, preferably at least 60%, more preferably at least 70%, still more preferably at least 80%, yet more preferably at least 90%, and, yet still more preferably at least 95%, 96%, 97%, 98% or even 99%, of said cells with different phenotype.

As used herein, "CCR7" relates to a chemokine receptor coupled to protein G involved in the migration of memory T lymphocytes towards inflamed tissues and in the maturation of the dendritic cells. The human CCR7 sequence corresponds with sequence number P32248 of the Uniprot database at 27^{th} December 2010. The term "CCR7" includes native protein as well as the functionally equivalent variants thereof.

As used herein, "CD14" relates to a membrane receptor expressed in myelomonocytic cells the function of which is for receiving lipopolysaccharide (LPS) complex and lipopolysaccharide-binding protein (LBP). The human CD14 sequence corresponds with sequence number P08571 of the Uniprot database at 27^{th} December 2010. The term "CD14" includes native protein as well as the functionally equivalent variants thereof.

As used herein, "CD80", also known as B7.1, relates to a membrane receptor expressed in activated B lymphocytes, activated T lymphocytes and macrophages. The human CD80 sequence corresponds with sequence number P33681 of the Uniprot database at 27^{th} December 2010. The term "CD80" includes native protein as well as the functionally equivalent variants thereof.

As used herein, "CD83" relates to a maturation marker of dendritic cells. The human CD83 sequence corresponds with sequence number Q01151 of the Uniprot database at 27^{th} December 2010. The term "CD83" includes native protein as well as the functionally equivalent variants thereof.

As used herein, "CD86", also known as B7.2 relates to a protein expressed in the antigen presenting cells providing co-stimulatory signals needed for activating T lymphocytes and survival. The human CD86 sequence corresponds with sequence number P42081 of the Uniprot database at 27^{th} December 2010. The term "CD86" includes native protein as well as the functionally equivalent variants thereof.

"Dendritic cells" or "DCs" are very specialized antigen presenting cell (CPA) with capacity for initiating the primary immune responses.

As used herein, the term "tolerogenic dendritic cells" or "tolerogenic DCs" relates to DCs with capacity for inducing tolerance, i.e., having low capacity for activating effector T lymphocytes, but having great capacity for inducing and activating regulatory T lymphocytes.

As used herein, a "cytokine" is a protein that can be secreted by different types of leukocytes with structural and functional differences which participate in intercellular communication. Cytokines can act on the same leukocytes or on other cells and they all act through constitutive or inducible receptors. In a particular embodiment, said cytokine is selected from interleukin 4 (IL-4), granulocyte-macrophage colony-stimulating factor (GM-CSF), combinations thereof and functionally equivalent variants thereof.

As used herein, a "proinflammatory cytokine" is a cytokine acting like an immunomodulator agent which promotes the systemic inflammation; illustrative examples of proinflammatory cytokines include IL-1β, IL-6, TNF-α, PGE2, etc. In a particular embodiment said proinflammatory cytokine is selected from the group consisting of IL-1β, IL-6, TNF-α, PGE2 and combinations thereof.

As used herein, "immune-mediated inflammatory disease" or "IMID" is understood as the disease caused because the immune system starts an inappropriate immune response resulting in damage of the own tissues and organs. In a particular embodiment, said IMID is selected from systemic sclerosis, alopecia areata, ankylosing spondylitis, autoimmune cardiomyopathy, autoimmune hemolytic anemia, autoimmune hepatitis, autoimmune disease of the inner ear, autoimmune lymphoproliferative syndrome, autoimmune peripheral neuropathy, autoimmune pancreatitis, autoimmune polyendocrine syndrome, autoimmune progesterone dermatitis, idiopathic thrombocytopenic purpura, autoimmune uveitis, celiac disease, cold agglutinin disease, Crohn's disease, ulcerative colitis, dermatomyositis, diabetes s mellitus type I, eosinophilic fasciitis, gastrointestinal pemphigoid, Goodpasture's syndrome, Graves' disease, Guillain-Barré syndrome, Hashimoto's encephalopathy, Hashimoto's thyroiditis, lupus erythematosus, Miller-Fisher syndrome, mixed connective tissue disease, myasthenia gravis, narcolepsy, pemphigus vulgaris, pernicious anemia, polymyositis, primary biliary cirrhosis, primary sclerosing cholangitis, psoriasis, psoriatic arthritis, relapsing polychondritis, rheumatoid arthritis, Sjögren's syndrome, temporal arteritis, ulcerative colitis, vasculitis, Wegener's granulomatosis and multiple sclerosis. In a particular embodiment, said IMID is Crohn's disease, a chronic autoimmune disease in which the immune system of the individual attacks his own intestine producing inflammation; although the disease can manifest on any place of the digestive tract the frequently affected part is the ileum or the final section of the small intestine,.

As used herein, a "steroid glucocorticoid" is a steroid hormone characterized by its capacity for binding to the cortisol receptor. Non-limiting illustrative examples of synthetic steroid glucocorticoids include dexamethasone, fludrocortisone, prednisone, triamcinolone, betamethasone, aldosterone, etc.; In a particular embodiment, steroid glucocorticoid is dexamethasone [9-fluoro-11β, 17, 21-trihydroxy-16a-methylpregna-1,4-diene-3,20-dione]. The term "dexamethasone" includes the functionally equivalent variants thereof.

As used herein, "GM-CSF" relates to granulocyte-macrophage colony-stimulating factor secreted by macrophages, T lymphocytes, mast cells, endothelial cells and fibroblasts. This factor stimulates the stem cells differentiating them into granulocytes and monocytes. The human GM-CSF sequence corresponds with sequence number P04141 of the Uniprot database at 27^{th} December 2010. The term "GM-CSF" includes the native protein as well as the functionally equivalent variants thereof.

As used herein, the term "hemoderivative" of peripheral blood" includes all those that are derived from peripheral blood which do not present their entire components, for example, a product resulting from an apheresis process, buffy coat, platelet concentrates, etc.

As used herein, "IL-1β" relates to interleukin 1 beta, a cytokine produced by activated macrophages; IL-1β is an important mediator of inflammatory response and it is involved in a variety of cell activities including cell proliferation, cell differentiation and apoptosis. The human IL-1β sequence corresponds with sequence number P01584 of the Uniprot database of 27 December 2010. The term "IL-1β" includes the native protein as well as the functionally equivalent variants thereof.

As used herein, "IL-4" relates to interleukin 4, a cytokine that stimulates the IgE antibody production and TH2 proliferation and differentiation, antagonizes the macrophage activation effects of interferon (IFN) and blocks the de *novo* synthesis of cytokines. The human IL-4 sequence corresponds with sequence number P05112 of the Uniprot database at 27^{th} December 2010. The term "IL-4" includes the native protein as well as the functionally equivalent variants thereof.

As used herein "IL-6" relates to interleukin 6, a glycoprotein secreted by macrophages, T cells, endothelial cells and fibroblasts. Its release is induced by interleukin 1 (IL-1) and is increased in response to TNFα. The human IL-6 sequence corresponds with sequence number P05231 of the Uniprot database at 27^{th} December 2010. The term "IL-6" includes the native protein as well as the functionally equivalent variants thereof.

An "effector T lymphocyte" is a cell activated by the recognition of an antigen on its TCR which entails phenotypic changes (they increase the molecule expression in the membrane thereof and the secretion of cytokines facilitating the maturation of T lymphocytes) and changes in its function of performing a specific and efficient immune response.

As used herein a "regulatory T lymphocyte" is a cell from a specialized sub-population of T lymphocytes which acts by suppressing the activation of immune system, thus maintaining the homeostasis of this system and favoring the tolerance towards self antigens.

As used herein, the term "marker" relates to a protein distinguishing a cell (or group of cells) from another cell (or group of cells). For example, a protein expressed on the surface of precursor cells but not on other cells of a cell population acts as protein marker for the precursor cells. Normally, the marker is a cell surface antigen such that antibodies that attach to the protein marker can be used in cell selection methods, for example, for producing a cell population rich in cells which express the protein marker.

As used herein, "MHCI" or major histocompatibility complex I relates to a set of genes encoding glycoproteins with structure similar to immunoglobulin expressed on the surface of all nucleated cells the main function of which is presenting intracellular antigenic peptides to the cytotoxic T lymphocytes (CD8+).

As used herein, "MHCII" or major histocompatibility complex II relates to a set of genes encoding glycoproteins with immunoglobulin structure expressed in antigen presenting cells where they present processed extracellular antigenic peptides to the helper T lymphocytes (CD4+).

As used herein, "monocyte" is understood as a leukocyte of agranulocyte type characterized by having a kidney-shaped nucleus. It represents 2% to 9% of the total leukocytes circulating through blood vessels.

As used herein, "PGE2" relates to prostaglandin E2 [9-oxo-11α,15S-dihydroxy-prosta-5Z,13E-dien-1-oic acid]. The term "PGE2" includes native prostaglandin as well as the functionally equivalent variants thereof.

As used herein, "peripheral blood" relates to blood circulating through the body of a subject.

AS used herein, the term "subject" includes any animal having blood circulation, preferably, a mammal, more preferably, a primate, and, yet more preferably, a human being.

As used herein, "TNF-α" relates to tumor necrosis factor alpha; its stimulation is related to other mediator cells such as IL-1 and bacterial endotoxins. TNF-α plays different functions in different organs, for example, the activation of the production of other mediators such as IL-1, IL-6, etc. The TNF-α sequence corresponds with sequence number P01375 of the Uniprot database at 27^{th} December 2010. The term "TNF-α" includes the native protein as well as the functionally equivalent variants thereof.

As used herein, the term "functionally equivalent variant" relates to a molecule conserving a function or activity of another different molecule. Variants can be prepared from a given molecule by means of chemically modifying said molecule by using conventional methods known by the person skilled in the art. Naturally, said variant will bind to the receptor and its intracellular signaling activity is similar to the corresponding native molecule. A person skilled in the art will understand that the method suitable for determining whether a variant of a molecule is functionally equivalent to another will depend on the function, activity or effects of said molecule with which the functionally equivalent variant is to be compared.

The term "pharmaceutically acceptable carrier" relates to a carrier that must be approved by the regulatory agency of the federal or state government, or listed in the United States Pharmacopeia or the European Pharmacopeia or other pharmacopeia generally acknowledged for the use thereof in animals, and more specifically in humans.

As used herein, a "fat soluble vitamin" is an essential substance for cell functioning, normal growth and development, soluble in organic solvents, fats and oils. Illustrative examples of fat soluble vitamins include vitamins A, D, E, K and F. In a particular and preferred embodiment, said fat soluble vitamin is vitamin A or retinol [3,7-dimethyl-9-(2,6,6-trimethylcyclohexen-1-il)nona-2,4,6,8-tetraen-1-ol] or a functionally equivalent variant thereof.

### Method of the invention

The inventors of this invention have developed a method for obtaining mature tolerogenic DCs from monocytes (Example 1) having very potent tolerogenic properties such as shown in Example 2 in which it is shown that said tolerogenic DCs secrete IL-10, an interleukin involved in the attenuation and generation of immune response, and they further have a reduced allogeneic response such as shown in Example 3. Therefore, said tolerogenic DCs provided by the present invention can be used in the treatment of immune-mediated inflammatory diseases (IMIDs).

Therefore, in one aspect, the invention relates to a method for obtaining tolerogenic dendritic cells (DCs), hereinafter method of the invention, which comprises:
a) culturing monocytes originating from a peripheral blood sample of a subject or from a hemoderivative thereof in a culture medium comprising a cytokine, for a time period comprised between 2 and 3 days;
b) culturing the cells resulting from step a) in a culture medium comprising a cytokine, a fat soluble vitamin and a steroid glucocorticoid for a time period of 4 days to obtain mature DCs;
c) culturing said mature DCs resulting from step b) in a culture medium comprising a proinflammatory cytokine to obtain tolerogenic DCs; and
d) isolating said tolerogenic DCs.

Briefly, the monocytes can be obtained from peripheral blood of a subject or from a hemoderivative thereof by means of conventional methods widely known by the person skilled in the art, for example, by means of adherent method, gradient sedimentation method, use of silicon colloidal particle, flow cytometry, among others [Larry M. Wahl et al., Curr Protoc Immunol. 2006 Jan; Chapter 7: Unit 7.6A]. Said monocytes are cultured in a suitable culture medium comprising a cytokine for a time period comprised between 2 and 3 days under suitable conditions [step a)]. In a particular embodiment, said cytokine is selected from the group consisting of cytokines IL-4, GM-CSF and combinations thereof. As mentioned previously, the terms IL-4 and GM-CSF include the functionally equivalent variants thereof. Under these conditions, the cells resulting from step a) present an intermediate phenotype mainly characterized by the loss of molecule CD14 expression.

Subsequently, the cells resulting from step a) are cultured [step b)] in a culture medium comprising a cytokine, a fat soluble vitamin and a steroid glucocorticoid for a time period of 4 days under suitable conditions known by the person skilled in the art which allow obtaining mature DCs. In a particular embodiment, said cytokine is selected from the group consisting of cytokines IL-4, GM-CSF and combinations thereof. As mentioned previously, the terms IL-4 and GM-CSF include the functionally equivalent variants thereof. In a particular embodiment, said fat soluble vitamin is vitamin A. Likewise, in a particular embodiment, said steroid glucocorticoid is dexamethasone. As mentioned previously, the terms IL-4, GM-CSF, vitamin A and dexamethasone include the functionally equivalent variants thereof. Under these conditions, the cells resulting from step b) become mature DCs.

Next, the mature DCs resulting from step b) are cultured [step c)] in a culture medium comprising a proinflammatory cytokine under suitable conditions for obtaining tolerogenic DCs; said conditions include culturing said mature DCs for a time period comprised between 24 and 48 hours. In a particular embodiment, said proinflammatory cytokine is selected from the group consisting of IL-1β, IL-6, TNF-α, PGE2 and mixtures thereof. As mentioned previously, the terms IL-1β, IL-6, TNF-α and PGE2 include the functionally equivalent variants thereof. Under these conditions, the cells resulting from step c) become tolerogenic DCs which can be isolated [step d)] by conventional methods known by the person skilled in the art.

More specifically, in a particular embodiment, the method of the invention comprises:
a) culturing monocytes originating from a peripheral blood sample of a subject or from a hemoderivative thereof in a culture medium comprising a cytokine selected from IL-4, GM-CSF and combinations thereof for a time period comprised between 2 and 3 days;
b) culturing the cells resulting from step a) in a culture medium comprising (i) a cytokine selected from IL-4, GM-CSF, and combinations thereof, (ii) vitamin A and (iii) dexamethasone for a time period of 4 days to obtain mature DCs,
c) culturing said mature DCs resulting from step b) in a culture medium comprising a proinflammatory cytokine selected from the group consisting of IL-1β, IL-6, TNF-α, PGE2 and combinations thereof for a time period comprised between 24 and 48 hours to obtain tolerogenic DCs, and
d) isolating said tolerogenic DCs.

### Tolerogenic DCs of the invention

In another aspect, the invention relates to a tolerogenic DC, hereinafter tolerogenic DC of the invention that can be obtained according to the method of the invention.

The tolerogenic DC of the invention is characterized in that it shows an increased level of CD14 marker in comparison with the level of said CD14 marker in a mature DC obtained in step b) of the method of the invention.

In the context of the present invention, the level of a marker is said to be "increased" when the level of said marker in a determined cell (e.g., in a tolerogenic DC of the invention) is elevated with respect to the level of said marker in another reference cell (e.g., the mature DCs, such as those obtained in step b) of the method of the invention). Thus, according to the present invention, it is considered that the level of a determined marker is increased when said level is incremented by at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or even more, with respect to the level of said marker in relation to the level of the marker in a reference cell (e.g., the mature DC obtained in step b) of the method of the invention).

Alternatively, the tolerogenic DC of the invention is characterized in that it shows a reduced level of the CD80, CD83 and/or CCR7 markers in comparison with the levels of said markers in a mature DC obtained in step b) of the method of the invention. In a particular embodiment, the tolerogenic DC of the invention is characterized in that it shows a reduced level of the 3 CD80, CD83 and CCR7 markers in comparison with the levels of said markers in a mature DC obtained in step b) of the method of the invention.

In the context of the present invention, the level of a marker is said to be "reduced" when the level of said marker in a determined cell (e.g., in a tolerogenic DC of the invention) is diminished with respect to the level of said marker in another reference cell (e.g., the mature DCs, such as those obtained in step b) of the method of the invention). Thus, according to the present invention, it is considered that the level of a determined marker is reduced when said level is diminished by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least 85%, at least 90%, at least 95%, at least 100%, at least 110%, at least 120%, at least 130%, at least 140%, at least 150% or even more with respect to the level of said marker in relation to the level of the marker in a reference cell (e.g., the mature DC obtained in step b) of the method of the invention).

In a particular embodiment, the tolerogenic DC of the invention is further characterized in that the CD86, MHCI and MHCII markers are maintained at levels similar to the levels of said markers in a mature DC obtained in step b) of the method of the invention.

In the context of the present invention, the level of a marker is said to be "maintained" at a level similar to another level when there is no significant difference between the value of the level of a marker in a determined cell (e.g., in a tolerogenic DC of the invention) and the value of the level of said marker in another reference cell (e.g., the mature DCs, such as those obtained in step b) of the method of the invention).

In a particular embodiment, the tolerogenic DC of the invention is characterized in that it shows:
i) an increased level of CD14 marker in comparison with the level of said CD14 marker in a mature DC obtained in step b) of the method of the invention; and
ii) a reduced level of the CD80, CD83 and/or CCR7 markers in comparison with the levels of said markers in a mature DC obtained in step b) of the method of the invention.

In a particular embodiment, the tolerogenic DC of the invention is characterized in that it shows:
i) an increased level of CD14 marker in comparison with the level of said CD14 marker in a mature DC obtained in step b) of the method of the invention; and
ii) a reduced level of CD80, CD83 and CCR7 markers in comparison with the levels of said markers in a mature DC obtained in step b) of the method of the invention.

Obviously, the tolerogenic DC of the invention can further contain other cell markers characteristic of said tolerogenic DCs of the invention.

The detection of said markers and the subsequent isolation of the cells expressing said markers, i.e., the isolation of the tolerogenic DCs of the invention, can be performed by means of any method which allows the separation of cells according to a given phenotype feature.

In a particular embodiment, in the cell isolation and/or identification assays, the cell population is contacted with a specific labeled or non-labeled reagent depending on whether the assay is performed by means of a direct or indirect detection method, respectively. As used herein, a "specific reagent" relates to a member of a specific binding pair, for example, binding pairs formed by antigens and antibodies, complementary nucleotide sequences as well as peptide ligand pairs and their receptor. The specific binding pairs include analogues, fragments and derivatives of a specific member of the binding pair.

The use of antibodies as affinity reagents is of particular interest. Specific monoclonal or polyclonal antibody production is known by the persons skilled in the art. In a specific embodiment, in cell population separation or identification assays, labeling can be performed with the suitable antibodies; to that end, tags, for example, magnetic particles, biotin and fluorochromes, among others can be used, which will allow the identification or separation of the cell type that the antibody has bound to. Thus, cell population analysis by means of flow cytometry, for example, allows using different antibodies labeled with fluorochromes emitting a different wavelength in one and the same sample. Therefore, the specific profile of the cell population for these surface markers can be known as well as a separation by the set of markers used can carried carried out.

The separation of the populations having the phenotype of interest can be carried out by means of affinity separation techniques, which include: magnetic separation (by using magnetic particles coated with specific antibodies), affinity chromatography, cytotoxic agents bound to monoclonal antibodies or used together with monoclonal antibodies, and "panning" with the antibody associated with a solid support, as well as by means of other suitable techniques. A more accurate separation can be obtained by means of flow cytometry, a technique which allows separating cell populations depending on the staining intensity, together with other parameters such as the cell size and the cell complexity.

Flow cytometry is a cell analysis technique which involves measuring the light and fluorescence dispersion characteristics of the cells as they are passed through a laser beam. In flow cytometry analysis, cells are placed in the presence of a compound that binds specifically to said marker, such as an antibody, for example, and the cells are classified depending on the compound. If the compound is labeled with fluorescence, it strictly concerns flow cytofluorometry, those known as "cytometries" or "FACS" (Fluorescence Analyzer Cell Sorter). Flow cytometries can analyze particles depending on their fluorescence and size. Those known as separators or "sorters" can also purify populations depending on determined characteristics.

In flow cytometry, in order for a marker to be considered positive, the specific signal observed must be stronger than the intensity of the background signal, it must typically be at least 10%, preferably 20%, 30%, 40%, 50%, 60%, 70%, 80%, 90%, or even 100% higher than the intensity of the background signal, by using conventional methods and apparatus. Background signal is defined as the intensity of the signal given by a non-specific antibody of the same isotype as the specific antibody used for detecting the surface marker in conventional FACS analysis.

In another aspect, the invention relates to an isolated cell population, hereinafter, "cell population of the invention", comprising tolerogenic DCs of the invention. The cell population of the invention can be preserved for a long period in a suitable medium compatible with the tolerogenic DCs of the invention, for example, isotonic solutions, optionally supplemented with serum; cell culture media or, alternatively, a solid, semisolid, gelatinous or viscous support medium as mentioned below.

In another aspect, the invention relates to a cell composition in which at least 50% of the cells are tolerogenic DCs of the invention. In a particular embodiment, said cell composition is a composition of tolerogenic DCs of the invention in which at least 60%, preferably 70%, more preferably 80%, still more preferably 90%, still more preferably 95%, and yet still more preferably 100% of the cells are tolerogenic DCs of the invention.

Said cell composition can contain a medium containing the cells; said medium must be compatible with said cells particularly with the tolerogenic dendritic cells of the invention present in said composition, for example, isotonic solutions optionally supplemented with serum; cell culture media or, alternatively, a solid, semisolid, gelatinous or viscous support medium as mentioned below.

### Applications of the tolerogenic DCs of the invention

In another aspect, the invention relates to a pharmaceutical composition comprising at least one tolerogenic DC of the invention, or a cell population of the invention, or a cell composition of the invention, and a pharmaceutically acceptable carrier, such as a carrier approved by a regulatory agency of the federal or state government, or listed in the United States Pharmacopeia or the European Pharmacopeia or other pharmacopeia generally acknowledged for the use thereof in animals, and more specifically in humans.

As used herein, the term "carrier" includes diluents, coadjuvants, excipients or vehicles whereby the tolerogenic DCs of the invention, the cell population of the invention or the cell composition of the invention must be administered. Obviously, said carrier must be compatible with said tolerogenic DCs. Non-limiting illustrative examples of said carrier include any physiologically compatible carrier, for example, isotonic solutions (e.g., 0.9% NaCl sterile saline solution, phosphate buffered saline (PBS) solution, Ringer-lactate solution, etc.) optionally supplemented with serum, preferably with autologous serum; cell culture media (e.g., DMEM, etc.); or, alternatively, a solid, semisolid, gelatinuos or viscous support medium such as collagen, collagen-glycosaminoglycan, fibrin, polyvinyl chloride, poly(amino acids) such as polylysine, polyornithine, etc., hydrogels, agarose, silicone dextran sulfate. Likewise, if desired, the support medium can, in specific embodiments, contain growth factors or other agents. If the support is a solid, semisolid, or gelatinous support, the tolerogenic DCs of the invention can be introduced into a liquid phase of the carrier which is subsequently treated such that it becomes a more solid phase.

The pharmaceutical composition of the invention, if desired, cal also contain, when necessary, additives to increase, control or give rise, in another manner, to the desired therapeutic effect of the tolerogenic DCs of the invention which comprise said pharmaceutical composition, and/or auxiliary substances or pharmaceutically acceptable substances such as buffering agents, surfactants, cosolvents, preservatives, etc. It is also possible to add metal chelates to stabilize the cell suspension. The stability of the tolerogenic DCs of the invention in liquid medium of the pharmaceutical composition of the invention can be improved by means of adding additional substances such as, for example, aspartic acid, glutamic acid, etc. Said pharmaceutically acceptable substances which can be used in the pharmaceutical composition of the invention are generally known by the persons skilled in the art and are normally used in the preparation of cell compositions. Examples of suitable pharmaceutical carriers are described, for example, in "Remington's Pharmaceutical Sciences", of E.W. Martin. Additional information about said carriers can be found in any manual of pharmaceutical technology (Galenic Pharmacy).

The pharmaceutical composition of the invention will contain a prophylactic or pharmaceutically effective amount of tolerogenic DCs of the invention, or of the cell population of the invention, or of the cell composition of the invention, preferably a substantially homogenous population of tolerogenic DCs of the invention to provide the desired therapeutic effect. In the sense used in this description, the term "therapeutic or prophylactically effective amount" relates to the amount of tolerogenic DCs of the invention, or of the cell population of the invention, or of the cell composition of the invention contained in the pharmaceutical composition of the invention which is capable of producing desired therapeutic effect and will generally be determined by, among other factors, the characteristics of said tolerogenic DCs themselves and the desired therapeutic effect. Generally, the therapeutically effective amount of said tolerogenic DCs of the invention that must be administered will depend on, among other factors, the characteristics of the subject himself, the seriousness of the disease, the dosage form, etc. For this purpose, the dose mentioned in this invention must only be taken into account as a guideline for the person skilled in the art, who must adjust this dose depending on the aforementioned factors. In a particular embodiment, the pharmaceutical composition of the invention is administered in a dose containing between approximately 1×10⁵ and approximately 1×10⁸ tolerogenic DCs of the invention per kilogram (kg) of body weight of the receiver, typically, about 10⁶-10⁷ DCs of the invention per inoculation, on a weekly, fortnightly or monthly basis, and with an inoculation range comprised between 3 and 6 inoculations per patient, such as described in the previously published clinical trials with DCs for the treatment of tumors (http://www.clinicaltrials.gov). In a specific embodiment, by way of non-limiting illustrative example the pharmaceutical composition of the invention can be administered as a single dose containing between approximately 1×10⁵ and approximately 10×10⁸ tolerogenic DCs of the invention per kg of body weight of the receiver, preferably between approximately 5×10⁵ and approximately 5×10⁷ tolerogenic DCs of the invention per kg of body weight of the receiver, more preferably between approximately 1×10⁶ and approximately 1×10⁷ tolerogenic DCs of the invention per kg of the body weight of the receiver depending on the aforementioned factors. The dose of tolerogenic DCs of the invention can be repeated depending on the status and evolution of the subject in temporal intervals of days, weeks or months that must be established by the specialist in each case.

The pharmaceutical composition of the invention will be formulated according to the selected method of administration. The pharmaceutical composition of the invention can be prepared in a gel or liquid dosage form, for example, in the form of suspension to be injected or perfused into a subject. Non-limiting illustrative examples include formulating the pharmaceutical composition of the invention in a sterile suspension with a pharmaceutically acceptable excipient such as an isotonic solution, for example, phosphate buffered saline (PBS) solution, or any other suitable pharmaceutically acceptable carrier for administration into a subject, for example, a human being through parenteral route, for example, through intradermal (i.d.) route, intraganglionar (i.g.) route, intraperitoneal (i.p.) route, intravenous (i.v.) route, subcutaneous (s.c.) route, etc., although other alternative administration routes are also possible. In a particular embodiment, the pharmaceutical composition of the invention is administered through intraperitoneal route because this route has been efficient in experimental studies and it confers the potential advantage of directing the injected DCs towards the lymph glands of the mesentery, which participate actively in intestinal immune response.

The administration of the pharmaceutical composition of the invention to the subject will be carried out by conventional means, for example, said pharmaceutical composition can be administered to said subject through parenteral route, for example, through i.d. route, i.g. route, i.p. route, i.v. route, s.c. route, etc., by using suitable devices such as syringes, catheters (a standard peripheral intravenous catheter, a central venous catheter or a pulmonary artery catheter, etc.), trocars, cannulas, etc. The cell flow can be controlled by sequentially inflating and deflating distal and proximal balloons located in the vasculature of the subject, thus creating temporary flow free areas that promote the therapeutic cell action. In all cases, the pharmaceutical composition of the invention will be administered using equipment, apparatuses and devices suitable for administering cell compositions known by the person skilled in the art.

As understood by a person skilled in the art, sometimes the direct administration of the pharmaceutical composition of the invention to the site wishing to benefit can be advantageous. Therefore, the direct administration of the pharmaceutical composition of the invention to the desired organ or tissue can be achieved by direct administration (e.g., through injection, etc.) on the outer surface of the affected organ or tissue by means of inserting a suitable device, e.g., a suitable cannula, by arterial or venous perfusion (including retrograde flow mechanisms) or by other means mentioned in this description or known in the technique.

The pharmaceutical composition of the invention, if desired, can be stored until its time of use by means of conventional processes known by the person skilled in the art. This pharmaceutical composition can also be stored together with additional medicaments useful in the treatment of diseases, in an active form comprising a combined therapy. For short term storage (less than 6 hours), the pharmaceutical composition of the invention can be stored at or below room temperature in a sealed container, complementing it or not with a nutrient solution. Medium term storage (less than 24 hours) is performed preferably at 2-8°C and the pharmaceutical composition of the invention will include a buffered iso-osmotic solution in a container made up of or covered with a material which prevents cell adhesion. Long term storage is preferably carried out by means of suitable cryopreservation and storage in conditions which promote the retention of cell function.

The pharmaceutical composition of the invention can be used in a combined therapy, as described above, useful in the prevention and/or treatment of the disease to be treated. Said additional compounds can form part of the same pharmaceutical composition or can be alternatively supplied in the form of a separate composition for the simultaneous or successive (sequential in time) administration with respect to the administration of the pharmaceutical composition of the invention. Another option consists of mixing whichever of said additional compounds in a same composition and administering them together.

In another additional aspect, the invention relates to the use of a tolerogenic DC of the invention, or of a cell population of the invention, or of a cell composition of the invention, or of a pharmaceutical composition of the invention, for the preparation of a medicament for the treatment of an immune-mediated inflammatory disease (IMID). In other words, in another additional aspect the invention relates to a tolerogenic DC of the invention, or to a cell population of the invention, or to a cell composition of the invention, or to a pharmaceutical composition of the invention for the use thereof in the treatment of an IMID.

In a particular embodiment, said IMID is selected from systemic sclerosis, alopecia areata, ankylosing spondylitis, autoimmune cardiomyopathy, autoimmune hemolytic anemia, autoimmune hepatitis, autoimmune disease of the inner ear, autoimmune lymphoproliferative syndrome, autoimmune peripheral neuropathy, autoimmune pancreatitis, autoimmune polyendocrine syndrome, autoimmune progesterone dermatitis, idiopathic thrombocytopenic purpura, autoimmune uveitis, celiac disease, cold agglutinin disease, Crohn's disease, ulcerative colitis, dermatomyositis, diabetes mellitus type I, eosinophilic fasciitis, gastrointestinal pemphigoid, Goodpasture's syndrome, Graves' disease, Guillain-Barré syndrome, Hashimoto's encephalopathy, Hashimoto's thyroiditis, lupus erythematosus, Miller-Fisher syndrome, mixed connective tissue disease, myasthenia gravis, narcolepsy, pemphigus vulgaris, pernicious anemia, polymyositis, primary biliary cirrhosis, primary sclerosing cholangitis, psoriasis, psoriatic arthritis, relapsing polychondritis, rheumatoid arthritis, Sjögren's syndrome, temporal arteritis, ulcerative colitis, vasculitis, Wegener's granulomatosis and multiple sclerosis. In a particular embodiment, said IMID is Crohn's disease.

The invention is illustrated below based on the following illustrative examples which are non-limiting to the scope of the invention.

### EXAMPLE 1

### Obtaining and characterizing tolerogenic dendritic cells

### 1.1 Obtaining tolerogenic DCs

### Materials

X-VIVO® 15 medium (BE04-418Q) (Lonza), medium with L-glutamine, gentamicin and phenol red.

20% human albumin (Grifols). Cytokines IL-1β, IL-4, IL-6, GM-CSF and TNF-α were produced by CellGenix (CellGro® GMP) in GMP conditions. Prostaglandin E2 Upjohn (Pfizer). Dexamethasone (Fortecortin®) Merck Farma. Vitamin A (Chiesi). PBS. DMSO (WAK-Chemie Medical GmbH), Histopaque-1077 (Ficoll) (Sigma), trypan blue (Merck). 0.9% sodium chloride solution for injection.

### Method

For obtaining the tolerogenic DCs of the invention, monocytes were isolated from a peripheral blood sample obtained from healthy donors (*buffy coats*). The samples were obtained from the Catalonia tissue blood bank (TBB) after receiving informed consent and being approved by the Ethic Committee of said centre. To that end, the product of the apheresis was mixed in a culture flask and 200 ml of sterile phosphate buffered saline (PBS) were added to dilute it. 12 ml of Ficoll were then distributed at room temperature in 6 50 ml tubes and the mixture of the apheresis with PBS was gently deposited on Ficoll so that, in the first place, 30 ml were added and it was then diluted up to 50 ml per tube. In this process it is important to ensure that the pipette is actuated with minimum strength and that it is left to slip over the wall of the tube, that must be inclined. The tubes were then centrifuged at 700 g for 20 minutes without speeding up or slowing down at a temperature of 20°C. The interphase that appeared after centrifugation which could be seen as a whitish ring in the upper part of the Ficoll was collected with a sterile Pasteur pipette and was placed in 50 ml tubes, adding cold sterile PBS to dilute it up to 50 ml.

It was then centrifuged again for 10 minutes at 500 g at 20°C. The supernatant was sucked out and the cell pellet was resuspended again in cold PBS. It was necessary to repeat this process until the supernatant from the washings was clean (this point varies among donors); this process intends to remove platelets, implicating the visual evaluation of the samples.

Once cleaned, the cells were counted in a volume of 50 ml of PBS including a ratio of 1:1 with trypan blue in order to enable excluding dead cells. Thus the number of cells obtained and their viability were determined. Finally, another centrifugation was performed to resuspend the mononuclear cell pellet from peripheral blood at a concentration of 12.5 millions of cells per ml with the X-VIVO ™ 15 culture medium and 2% patient serum.

8 ml of the resuspended cells were taken and were distributed in culture flasks of the type T-75. The culture flasks with the cells were placed in a cell culture incubator for 2 hours at 37 °C. This process allowed the specific adherence of monocytes to the plastic.

Cells not adhered to the plastic (supernatant) representing the remaining non-monocytes cells (mainly T and B lymphocytes, NK cells) described as PBLs (peripheral blood lymphocytes) were collected. These cells were cryopreserved (conveniently identified) and were used as preinoculation controls to monitor the immune response of the patient in response to tolerogenic DCs.

The adhered cells, monocytes, were cleaned with PBS at room temperature. To that end, the medium of the culture flask was collected, fresh medium was added, it was removed and finally 10 ml of PBS were added. This washing process was repeated approximately three times, although the purity can be controlled in microscope until there are no cells left in the supernatant.

Finally, 12 ml of the culture medium (X-VIVO® 15 + 2% human serum) and IL-4 (500 U/ml) and GM-CSF (800 U/ml) differentiation and growth factors were added to the monocytes (circulating precursors of the dendritic cells).

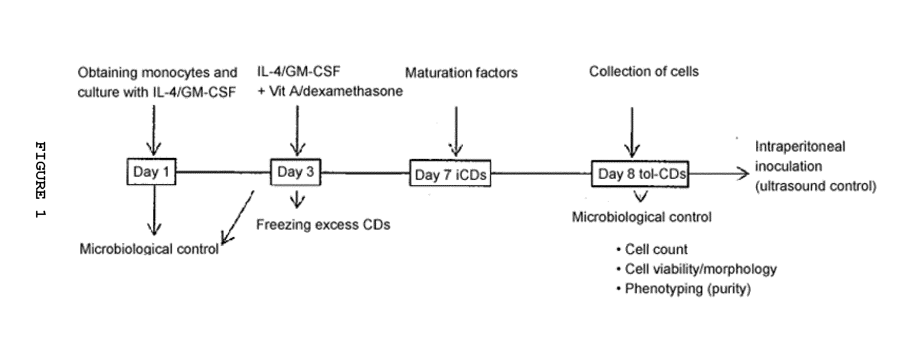

The culture flasks (conveniently identified) were left in the culture incubator at 37 °C with 5% CO₂ and 95% humidity for 2 days. On the third day, the culture medium was replaced by culture medium with IL-4 (500 U/ml), GM-CSF (800 U/ml), vitamin A 10⁻⁷ M and dexamethasone 10⁻⁶ M. On the seventh day immature dendritic cells (DCs) were obtained and the culture medium was substituted by a medium with maturation factors, IL-1β (10 ng/ml), IL-6 (20 ng/ml), TNF-α (20 ng/ml) and PGE2 (1 µg/ml). On the eight day the cells were collected and a microbiological control (Figure 1) was performed. At the end of this process tolerogenic DCs were obtained.

### 1.2 Phenotypic characterization of the tolerogenic dendritic cells

### Materials

Flow cytometer (BD FACScalibur). Monoclonal antibodies obtained from BD biosciences: anti-CD3, anti-CD19, anti-CD14, anti-CD80, anti-CD83, anti-CD86, anti-CCR7, anti-MHC I, anti-MHC II. Antibody against an irrelevant receptor not present in DCs, anti-KLH IgG1 obtained commercially from BD biosciences. Saline buffer (PBS). 96 well plates. Labeled secondary antibody, goat anti-mouse Ig antibody labeled with phycoerythrin (PE Goat anti-mouse Ig (Pharmingen)).

### Method

In the first place, to validate the process, the phenotype of the cells was analyzed by means of flow cytometry. To that end the presence of a series of membrane molecules having a close relationship with the activating or tolerogenic functionality of the DCs was analyzed. The expression of these molecules relates to the maturing stage of the DCs.

Phenotypic characterization of the previously obtained tolerogenic DCs (Example 1.1) was started from cells in suspension at a concentration of 1×10⁶ DCs/ml in saline buffer, 100 µl of the cells being distributed in each well of a 96 well plate. The plate was then centrifuged at 1,600 rpm for 5 minutes at 4°C. The supernatant was decanted and gently dried with a paper without disturbing the cell pellet. This cell pellet was resuspended in 200 µl of saline buffer and was centrifuged again and the supernatant was discarded. 25 µl (at an antibody concentration of 1 µl/ml diluted in saline buffer) of suitable monoclonal antibody depending on the molecule to be analyzed were added to each well and was incubated for 30 minutes at 4°C. The plate was centrifuged at 1,600 rpm for 5 minutes at 4°C, a washing was performed with 200 µl of saline buffer and it was centrifuged again in the same conditions. The wells were then incubated with 25 µl of the secondary antibody coupled to fluorochrome phycoerythrin (PE) for 30 minutes at 4°C and protected from light. The plate was centrifuged at 1,600 rpm for 5 minutes at 4°C, a washing was performed with 200 µl of saline buffer and it was centrifuged again in the same conditions. Finally, 200 µl of saline buffer (PBS) were added and the reading of the samples was performed by means of flow cytometer.

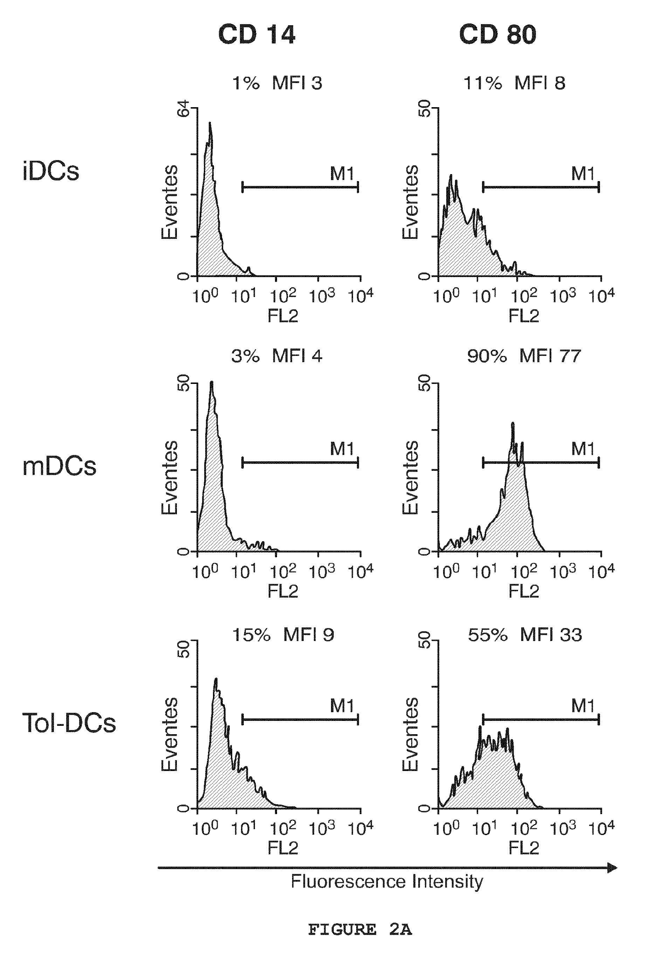

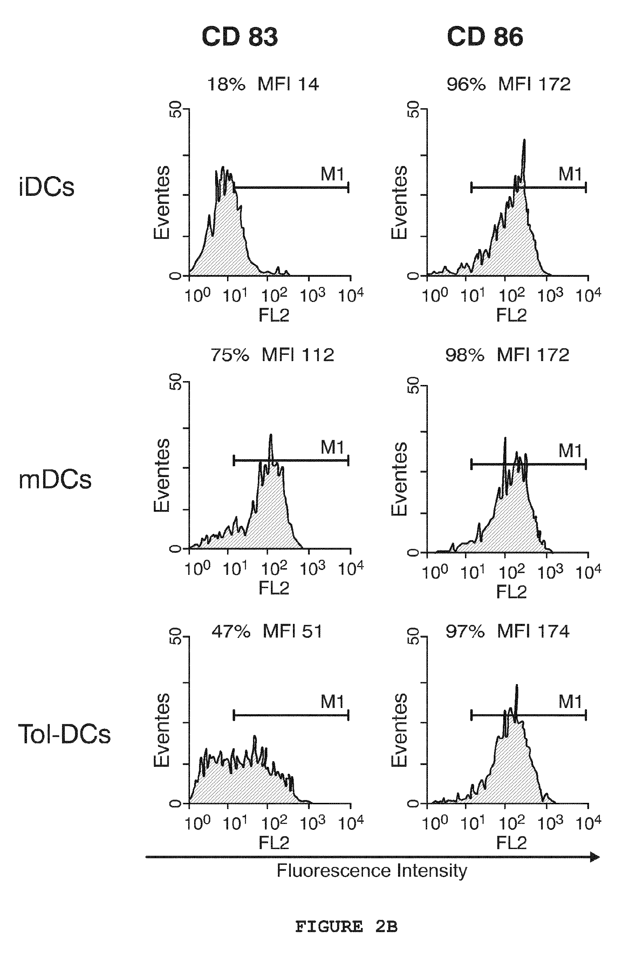

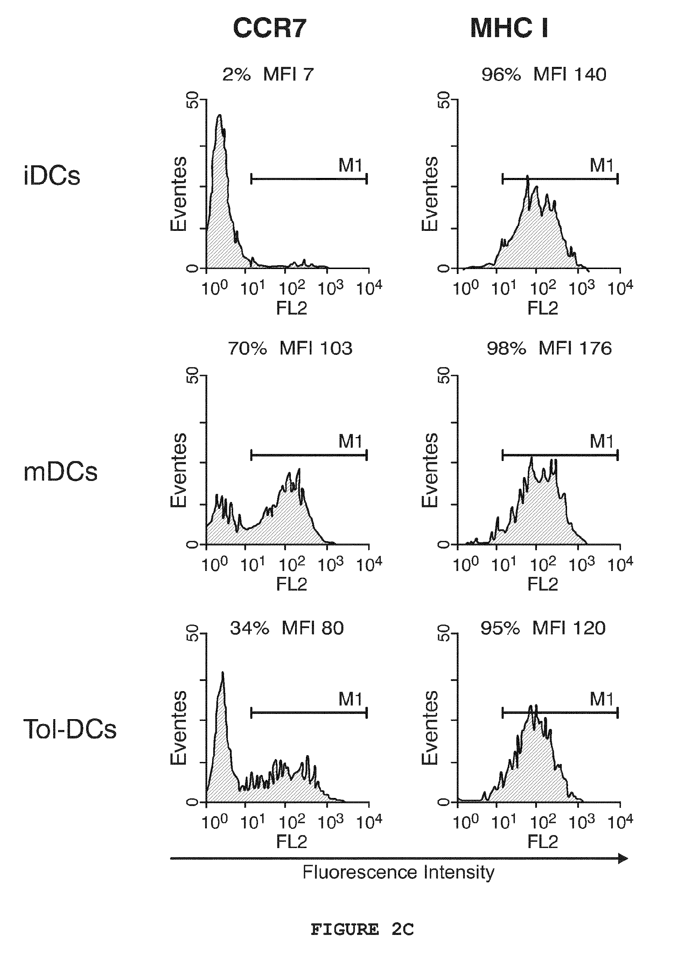

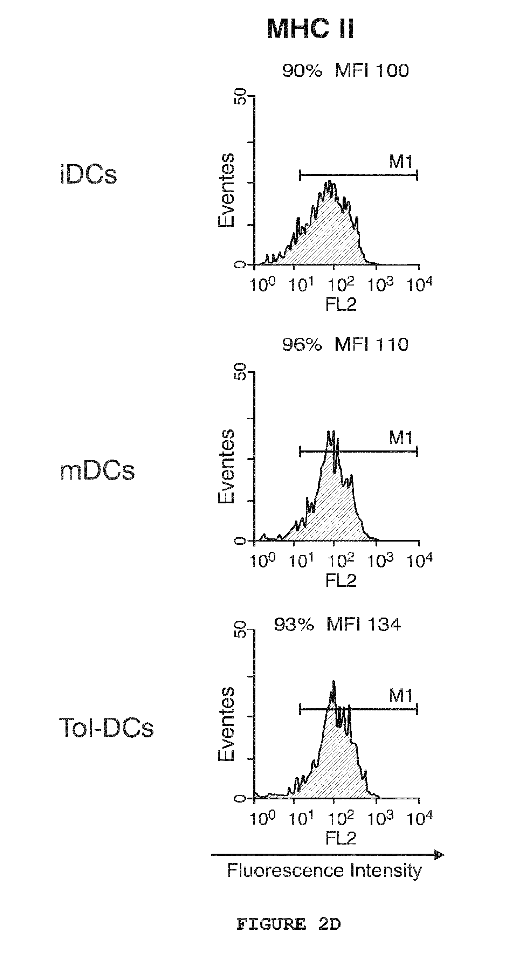

The results of the tolerogenic DCs were compared with those of the mature DCs to determine the markers that underwent a variation due to the presence of dexamethasone. An antibody, the anti-KLH antibody which did not recognize any molecule in the membrane, was included as the negative control of the experiment, furthermore antibodies (MHC class I and MHC class II) acting as positive control, since this molecule is always expressed on the membrane of all human cells. It was observed that the marker expression in tolerogenic DCs which increased the expression thereof in mature DCs (CD80, CD83 and CCR7) was much less, the CD83 marker especially underwent a significant variation relating to the tolerogenicity of the DCs. The CD14 marker seemed to be increased in the tolerogenic DCs, meanwhile CD86, MHCI and MHCII markers were constant when compared to mature DCs (Figure 2). Other markers for T and B lymphocytes have been included to evaluate the purity of the cells obtained and in all of the determinations the presence thereof has been less than 5% of the total cells.

### EXAMPLE 2

### Analysis of cytokine secretion of tolerogenic DCs

### Materials

Capture-detection antibody (depending on and being specific for each of the cytokines that were analyzed, e.g., IL-10, IL-12, etc.). Maxi-sorp 96 well plates (Nunc, USA). Streptavidin-HRP (horseradish peroxidase). Substrate (TMB: 3,3',5,5'-tetramethylbenzidine). Saline buffer (PBS). Diluent buffer (50 ml of saline buffer (PBS) + 25 µl of polysorbate (Tween®) [Sigma] + 0.05 g of albumin). Standard benchmark at known concentration. ELISA reader for detecting optical density.

### Method

After phenotypic characterization, the presence of cytokines (IL-10 and IL-12) secreted by tolerogenic DCs (Example 1) in the supernatants of activated cells was determined by means of the ELISA technique. The technique includes the inclusion of a positive control of known concentration validating the process in each determination.

The capture antibody diluted in saline buffer was incubated at a concentration of 2 µg/ml in plates of Maxi-sorp type overnight at 4°C to allow the binding of antibody to the plastic. On the following day three washings were performed with 200 µl of PBS-Tween (Sigma). The wells were then blocked with 200 µl of saline buffer with 2% albumin for 2 hours at room temperature. Once the time lapsed, 50 µl of samples to be analyzed were distributed in the wells in duplicate, diluted in the diluent, and incubated for 2 hours at room temperature. The dilution depends on the levels of cytokine to be detected and must be determined for each particular case. A standard curve at known concentrations, specifically at 1,000, 500, 250, 125, 62, 31 and 0 pg/ml was prepared. Three washings were performed with 200 µl of PBS-Tween (Sigma). Each well was then incubated with 50 µl of the corresponding antibody for 1 hour at room temperature. Three washings were again performed with 200 µl of PBS-Tween. Streptavidin-HRP diluted at 1/1000 in saline buffer in a final volume of 100 µl was then added for 30 minutes at room temperature. Three washings were performed with 200 µl of PBS-Tween. Finally, 100 µl of the substrate (TMB) were added until the color changed to blue, being detected with ELISA reader at 450 nm. The cytokine concentration was calculated from the standard curve representing a regression line of optical density versus known concentration.

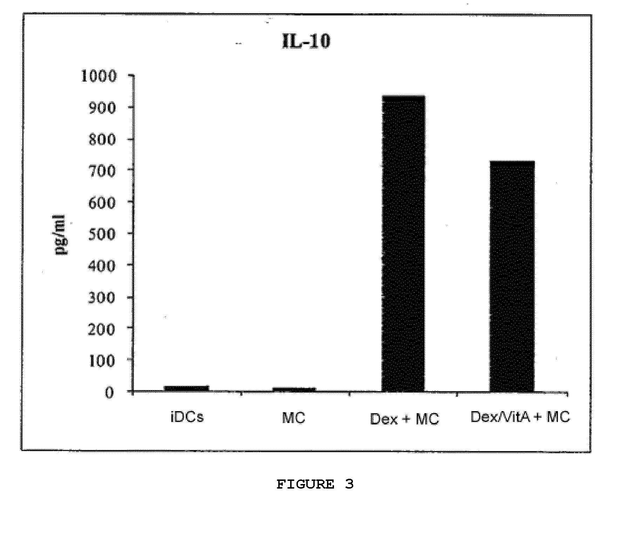

A significant increase of the levels of production of IL-10 was observed when the tolerogenic DCs were stimulated in the presence of dexamethasone with respect to tolerogenic DCs stimulated without dexamethasone (Figure 3). IL-10 is one of the most potent pro-inflammatory cytokines described, involved in the attenuation and generation of immune response tolerance.

An increase and/or a secretion of proinflammatory cytokines such as IL-12, TNF-α or IL-23 which are closely related to autoimmune processes or to chronic inflammation was not detected in any event.

### EXAMPLE 3

### Allogeneic response

### Materials

X-VIVO® 15 medium (BE04-418Q) (Lonza), medium with L-glutamine, gentamicin and phenol red supplemented with 2% human serum. U-shaped bottom 96 well plates (Costar, USA). Tritium labeled thymidine (Amersham, GE Healthcare, UK ref: TRK120). Scintillation counter model Packard 1600 TR.

### Method

The objective of determining allogeneic response is to determine the immunogenicity of the tolerogenic DCs produced (Example 1). It is based on the capacity of the T lymphocytes to proliferate in response to DCs coming from a different donor. This process is carried out in the research laboratory of the Gastroenterology Department of Hospital Clinic de Barcelona and in the radioactive laboratory of the same facility.

5×10³ DCs in 100 µl, obtained from DCs resuspended (5×10⁴ DCs/ml) in X-VIVO® 15 culture medium supplemented with 2% human serum, were distributed into each well of a plate of 96. 1×10⁵ PBLs (peripheral blood leukocytes) obtained from another donor not related with the donor from whom the DCs were obtained, were then added. The PBLs were distributed in 100 µl of medium, the final volume was therefore 200 µl. The culture plate was left for 4 days in an incubator at 37°C, 5% CO₂ and 95% humidity. After 4 days, tritium labeled thymidine with an activity of 1 µCi per well was added to each of the wells in a volume of 50 µl of culture medium (X-VIVO® 15 + 2% human serum). The cells were incubated for 16 hours with the radioactive thymidine allowing it to be incorporated into the DNA of the proliferating T lymphocytes. The radioactivity was detected with counter model Packard 1600 TR providing the radioactivity units (cpm) of each of the wells.

The maturation of DCs resulted in an increase of proliferation with respect to the presence of immature DCs (iDCs). This increase of proliferation with mature DCs is associated with the expression of costimulation molecules and histocompatibility molecules in the membrane of the DCs, as well as with the balance in the secretion of pro- and antiinflammatory cytokines (IL-10/IL-12).

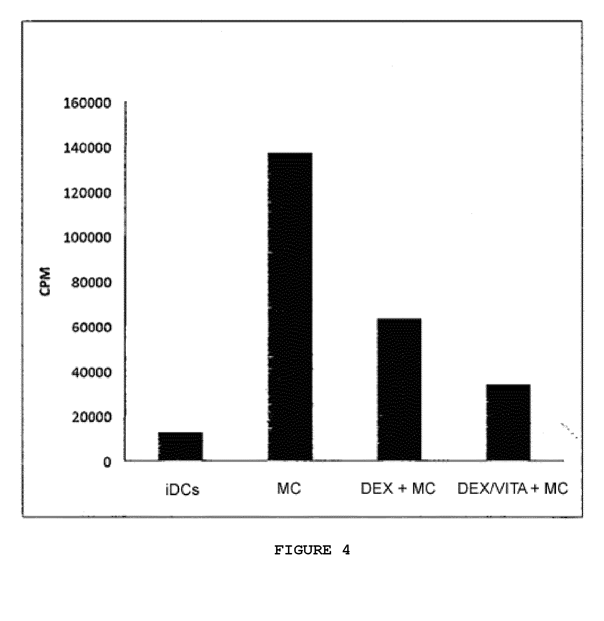

It was observed that the tolerogenic DCs (Example 1) had a reduced capacity for generating an allogeneic response compared to that of mature DCs. DCs cultured in the presence of dexamethasone have a reduced capacity in allogeneic response in comparison with mature DCs (Dex-MC or DexVitA-MC versus MC), the tolerogenic DCs induced proliferation being similar to that of the negative control with immature cells (iDCs) (Figure 4).

### EXAMPLE 4

### Administration protocol (Prophetic example)

### Clinical development plan

Since this is the first study with tolerogenic dendritic cells (DCs) in humans, it is practically impossible to provide a dose calculation in humans based on previous publications. The calculations are based on human studies for treatment with immunogenic DCs and on the experimental studies in which the efficacy of the treatment based on tolerogenic DCs has been shown.

The doses proposed for developing the study in phase I are the followings:
- Number of patients. 6 sequential groups with 2 patients in each group at increasing dose will be included, which represents a total of 12 patients.
- Dose. An inoculation regimen of one or three intraperitoneal injections will be carried out by means of ultrasound monitoring to inject the cells in the most suitable area with the tolerogenic DCs at the dose (per kg of body weight of the receiving person) detailed below:
   1^{st} group - 2x10⁶ tolerogenic DCs - single dose
   2^{nd} group - 5x10⁶ tolerogenic DCs - single dose
   3^{rd} group - 10x10⁶ tolerogenic DCs - single dose
   4^{th} group - 2x10⁶ tolerogenic DCs - three doses (week 0, 2 and 4)
   5^{th} group - 5x10⁶ tolerogenic DCs - three doses (week 0, 2 and 4)
   6^{th} group - 10x10⁶ tolerogenic DCs - three doses (week 0, 2 and 4)

The proposed regimen is based on the information available from previously published clinical trials with DCs for the treatment of tumors (according to data extracted from http://www.clinicaltrials.gov). The most common doses used varied between 5-15x10⁶ DCs up to a maximum of 1x10⁸ DCs per inoculation fortnightly and between an inoculation range of 3 and to the maximum of 6 inoculations per patient. It is generally accepted that the average dose is about 10⁷ DCs per inoculation, and that successive inoculations are necessary to achieve the desired therapeutic effect. These doses used have been well tolerated without serious associated adverse effects (the associated adverse effects are of type I or type II: slight fever, shivering, fatigue and allergy reaction in the inoculation area). The proposed doses are in the medium-low range of those used in the mentioned studies.

Another relevant aspect is the administration route. Various routes, mainly subcutaneous route or intradermal route, intravenous route and intraganglionar route have been used for inoculating DCs in previous studies. Differences in the frequency of adverse effects according to the administration route used have not been observed. In the present study, intraperitoneal route will be used because this is the route which has shown to be effective in experimental studies, and it confers the potential advantage of directing the injected DCs towards the mesenteric glands of the mesentery which participate actively in intestinal immune response.

## Claims

1. A method for obtaining tolerogenic dendritic cells (DCs) which comprises:
a) culturing monocytes obtained from a peripheral blood sample of a subject, or from a hemoderivative thereof, in a culture medium comprising a cytokine for a time period comprised between 2 and 3 days;
b) culturing the cells resulting from step a) in a culture medium comprising a cytokine, a fat soluble vitamin and a steroid glucocorticoid for a time period of 4 days to obtain mature DCs;
c) culturing said mature DCs resulting from step b) in a culture medium comprising a proinflammatory cytokine to obtain tolerogenic DCs; and
d) isolating said tolerogenic DCs.

2. The method according to claim 1, wherein said cytokines contained in the culture media of steps b) and c) are selected from the group formed by IL-4, GM-CSF and combinations thereof.

3. The method according to claim 1, wherein said fat soluble vitamin is vitamin A.

4. The method according to claim 1, wherein said steroid glucocorticoid is dexamethasone.

5. The method according to claim 1, wherein said proinflammatory cytokine is selected from the groupformed by IL-1β, IL-6, TNF-α, PGE2 and mixtures thereof.

6. The method according to any of claims 1 to 5, which comprises:
a) culturing monocytes obtained from a peripheral blood sample of a subject, or from a hemoderivative thereof, in a culture medium comprising a cytokine selected from IL-4, GM-CSF and combinations thereof, for a time period comprised between 2 and 3 days;
b) culturing the cells resulting from step a) in a culture medium comprising (i) a cytokine selected from IL-4, GM-CSF, and combinations thereof, (ii) vitamin A and (iii) dexamethasone for a time period of 4 days to obtain mature DCs,
c) culturing said mature DCs resulting from step b) in a culture medium comprising a proinflammatory cytokine selected from the group consisting of IL-1β, IL-6, TNF-α, PGE2 and combinations thereof for a time period comprised between 24 and 48 hours to obtain tolerogenic DCs, and
d) isolating said tolerogenic DCs.

7. A tolerogenic dendritic cell that can be obtained according to the method of any of claims 1 to 6.

8. The tolerogenic dendritic cell according to claim 7, **characterized in that** it shows an increased level of CD14 marker in comparison with the level of said CD14 marker in a mature DC obtained in step b) of the method of claim 1.

9. The tolerogenic dendritic cell according to claim 7 or 8, **characterized in that** it shows a reduced level of CD80, CD83 and/or CCR7 markers in comparison with the levels of said markers in a mature DC obtained in step b) of the method of claim 1.

10. The tolerogenic dendritic cell according to claim 7, **characterized in that** it shows:
(i) an increased level of the CD14 marker in comparison with the level of said CD14 marker in a mature DC obtained in step b) of the method of claim 1; and
(ii) a reduced level of CD80, CD83 and CCR7 markers in comparison with the levels of said markers in a mature DC obtained in step b) of the method of claim 1.

11. The tolerogenic dendritic cell according to any of claims 7 to 10, further **characterized in that** the CD86, MHCI and MHCII markers are maintained at levels similar to the levels of said markers in a mature DC obtained in step b) of the method according to claim 1.

12. An isolated cell population comprising tolerogenic dendritic cells according to any of claims 7 to 11.

13. A cell composition wherein at least 50% of the cells are tolerogenic dendritic cells according to any of claims 7 to 11.

14. A pharmaceutical composition comprising at least one tolerogenic dendritic cell according to any of claims 7 to 11, or a cell population according to claim 12, or a cell composition according to claim 13, and a pharmaceutically acceptable carrier.

15. A tolerogenic dendritic cell according to any of claims 7 to 11, or a cell population according to claim 12, or a cell composition according to claim 13, or a pharmaceutical composition according to claim 14 for its use in the treatment of an immune-mediated inflammatory disease (IMID).
